# EUROPÄISCHE PATENTSCHRIFT

(11) **EP 4 125 851 B1**
(45) Veröffentlichungstag und Bekanntmachung des Hinweises auf die Patenterteilung: **18.06.2025**
(21) Anmeldenummer: 20804417.2
(22) Anmeldetag: 27.10.2020
(51) Int. Cl.: A61K 31/198, A61K 31/26, A61K 31/352, A61P 7/02, A61P 31/14, A61P 35/04, A61K 33/34, A61K 33/30, A61K 33/32, A61K 31/10, A61K 31/13

(54) **ANTIVIRALE THROMBOSEHEMMENDE ZUSAMMENSETZUNG ZUR VERWENDUNG BEI DER BEHANDLUNG VON COVID-19 ENTHALTEND ARGININ UND THIOCYANAT**
ANTI-VIRAL ANTI-THROMBOTIC COMPOSITION FOR USE IN TREATING COVID-19 COMPRISING ARGININE AND THIOCYANATE
COMPOSITION ANTIVIRALE ANTI-THROMBOTIQUE POUR UTILISATION DANS LE TRAITEMENT DE COVID-19 COMPRENANT DE L'ARGININE ET DU THIOCYANATE

(43) Veröffentlichungstag der Anmeldung: 08.02.2023
(73) Patentinhaber: Weth, Gosbert, 97688 Bad Kissingen (DE)
(72) Erfinder: Weth, Gosbert, 97688 Bad Kissingen (DE)
(74) Vertreter: Gleim, Christian Ragnar
(86) Internationale Anmeldenummer: PCT/DE2020/100920
(87) Internationale Veröffentlichungsnummer: WO 2022/089677

(56) Entgegenhaltungen:
- EP-A1- 1 693 058
- EP-A2- 0 217 379
- WO-A2-2012/177847
- US-A1- 2009 226 538
- ALMEIDA JUNE D. ET AL: "The effect of sodium thiocyanate on virus structure", JOURNAL OF MEDICAL VIROLOGY, vol. 4, no. 4, 1 January 1979 (1979-01-01), US, pages 269 - 277, XP093201317, ISSN: 0146-6615, DOI: 10.1002/jmv.1890040405
- STANCIOIU FELICIAN ET AL: "A dissection of SARS-CoV2 with clinical implications (Review)", INTERNATIONAL JOURNAL OF MOLECULAR MEDICINE, vol. 46, no. 2, 10 June 2020 (2020-06-10), GR, pages 489 - 508, XP093076095, ISSN: 1107-3756, DOI: 10.3892/ijmm.2020.4636
- DATABASE GNPD [online] MINTEL; 9 April 2020 (2020-04-09), ANONYMOUS: "Raspberry Express Oral Care Foam", XP055820944, retrieved from https://www.gnpd.com/sinatra/recordpage/7468697/ Database accession no. 7468697
- SOUZA-COSTA D C ET AL: "l-Arginine attenuates acute pulmonary embolism-induced oxidative stress and pulmonary hypertension", NITRIC OXIDE: BIOLOGY AND CHEMISTRY, ACADEMIC PRESS, AMSTERDAM, NL, vol. 12, no. 1, 1 February 2005 (2005-02-01), pages 9 - 14, XP027215114, ISSN: 1089-8603, [retrieved on 20050201]
- EDSALL ET AL: "Effects of ions and neutral molecules on fibrin clotting.", JOURNAL OF BIOLOGICAL CHEMISTRY, vol. 191, no. 2, 1 August 1951 (1951-08-01), pages 735 - 756, XP055066734, ISSN: 0021-9258
- HRIZU DORINA ET AL: "In vivo and in vitro cytostatic effect of potassium sulfocyanate", ARCHIVES ROUMAINES DE PATHOLOGIE EXPERIMENTALE ET DEMICROBIOLOGIE, CARTIMEX, BUCHAREST, RO, vol. 32, no. 1, 1 January 1973 (1973-01-01), pages 155 - 158, XP009528430, ISSN: 0004-0037
- KLOK F A ET AL: "Incidence of thrombotic complications in critically ill ICU patients with COVID-19", THROMBOSIS RESEARCH, ELSEVIER, AMSTERDAM, NL, vol. 191, 10 April 2020 (2020-04-10), pages 145 - 147, XP086163213, ISSN: 0049-3848, [retrieved on 20200410], DOI: 10.1016/J.THROMRES.2020.04.013
- BURELA ALEJANDRA ET AL: "Chlorine dioxide and chlorine derivatives for the prevention or treatment of COVID-19: a systematic review", REVISTA PERUANA DE MEDICINA EXPERIMENTAL Y SALUD PÚBLICA, vol. 37, no. 4, 7 September 2020 (2020-09-07), pages 605 - 10, XP055820005, ISSN: 1726-4634, Retrieved from the Internet <URL:https://rpmesp.ins.gob.pe/index.php/rpmesp/article/viewFile/6330/3776> DOI: 10.17843/rpmesp.2020.374.6330

## Beschreibung

Die Erfindung bezieht sich auf antivirale thrombosehemmende Zusammensetzung zur Verwendung bei der Behandlung von Covid-19 mit Arginin als erstem Bestandteil dadurch gekennzeichnet, dass sie als einen zweiten Bestandteil Thiocyanate, auch Rhodanid genannt, enthält, und zwar als Kaliumthiocyanat oder als ein anderes Salz oder als ein Thiocyansäureester, gemäß der beigefügten Ansprüche.

Viren sind sehr viel älter als die Menschheit und sehr viel zahlreicher.

Die etwa 10¹² Zellen gesunder Menschen sind von 10¹⁴ Bakterien und 1 0¹⁶ Viren besiedelt. Die meisten davon sind in die normalen Körperfunktionen integriert. Es sind z.B. Viren, die Bakterien auf der Haut und im Darm steuern, und über das Mikrobiom wesentliche Beiträge zu einem funktionierenden Immunsystem leisten. (Karin Mölling, Direktorin des Institutes für medizinische Virologie, ETH Zürich, Supermacht des Lebens: Reisen in die erstaunliche Welt der Viren, C.H.Beck Verlag München, ISBN 978-3-406-66969-9, 18. November 2014)

In diese austarierte menschliche Balance von außen eindringende Viren können jedoch zu empfindlichen Störungen führen und im Extremfall letal wirken, wie z.B. die seit Dezember 2019 bekannten Coronaviren SARS-CoV-2.

Ähnliche, sehr empfindliche und ganz oft letale Störungen können auch einige der 10¹² menschlichen Zellen selbst verursachen, wenn sie zu Krebs entarten. Diese Erkrankung haben bereits in der Frühzeit alle Säugetiere erleiden müssen. Schon die Menschen-Affen wie beispielsweise der Australopithecus vor 2 bis 4,2 Millionen Jahren, hatten Krebs (M. Greaves: Krebs - der blinde Passagier der Evolution. Verlag Springer, 2002, ISBN 3-540-43669-3, S. 9.) Krebs ist eine Alterserkrankung des Zellwachstums, die mit dem Alter deutlich zunimmt.

Die Lebensdauer der menschlichen Zellen beträgt rund 5 Tage bei Schleimhäuten im Darm, 14 Tage bei Hautzellen, 120 Tage bei roten Blutkörperchen, 300 bis 500 Tage bei Leberzellen bis hin zu 10 Jahren bei Knochenzellen. In jeder Sekunde sterben im menschlichen Körper rund 50 Millionen Zellen ab und müssen abtransportiert werden. Ebenso viele entstehen neu.

Dabei kann es zu Krebs kommen, nämlich der unkontrollierten Vermehrung und dem wuchernden Wachstum von Zellen, die einen malignen (bösartigen) Tumor bilden. Entartete Zellen können sich auch absiedeln, im Blutkreislauf mitschwimmen und von dort aus in gesundes Gewebe eindringen, um Metastasen zu bilden.

Erst neuerdings wird beachtet, dass in sehr vielen Fällen der finale und letztlich letale Wirkungsablauf von Viren und in ähnlicher Weise der tödliche Einfluss von frei vagabundierenden Tumorzellen Lungenembolien sind, die in sehr kurzer Zeit ein Rechtsherzversagen verursachen, wodurch der Blutkreislauf zusammenbricht, so dass sämtliche Körperorgane versagen. Die eigentliche Todesursache ist also die Bildung von Thromben. Sie setzen sich auch in den Kapillaren der Lunge fest, wodurch der Gasaustausch in den betroffenen Alveolarzellen blockiert wird, so dass die Sauerstoffversorgung des Organismus eingeschränkt wird. Diesem Mangel versucht das Herz durch eine immer weiter ansteigende Pumpleistung entgegenzuwirken, die durch Überlastung der rechten Herzkammer in einem Stillstand mündet.

Die ursächlichen Viren sowie die unkontrolliert wachsenden Tumorzellen werden grundsätzlich vom Immunsystem versucht aufzuspüren und zu bekämpfen. Schon Louis Pasteur vermutete, dass Tumore durch eine Immunreaktion schrumpfen oder verschwinden können, was erst 1957 von Thomas und Burnet genauer beschrieben wurde. Da Tumorzellen aber in vielerlei Hinsicht normalen Körperzellen gleichen, sind die Unterschiede und damit die Abwehrmaßnahmen nicht immer ausreichend, um den Tumor zu kontrollieren. 18 (M. Burnet: Cancer; a biological approach. I. The processes of control. In: Br Med J. Band 1(5022), 1957, S. 779-786. PMID 13404306; PMC 1973174 ). Auch Viren können nicht immer oder nur in zu geringer Anzahl vom Immunsystem identifiziert und bekämpft werden.

Unterstützende Therapien gegen die letalen Thromben können sich direkt gegen die Thromben und/oder gegen die Beweglichkeit der Tumorzellen und/oder gegen das Eindringen und Replizieren von Viren richten. Eine kausale Behandlung von viralen Infektionskrankheiten wird dadurch sehr erschwert, dass Viren keinen eigenen Stoffwechsel haben. Virostatika, die die Vermehrung von Viren hemmen, werden vor allem für solche Infektionen eingesetzt, bei denen das Immunsystem des Patienten alleine nicht zur Eradikation des Virus in der Lage ist. Da die Vermehrung der Viren im Inneren von normalen Zellen stattfindet und sich dort sehr eng an die zentralen biochemischen Zellmechanismen ankoppelt, müssen die in Frage kommenden antiviralen Wirkstoffe entweder das Eindringen der Virionen in die Wirtszellen verhindern oder in den Zellstoffwechsel zum Nachteil der Virusvermehrung eingreifen. Von außen eingebrachte, zusätzliche Wirkstoffe für ein gezieltes Abtöten von bereits in den Körper eingedrungenen Viren sind höchst problematisch, weil sie für den Körperstoffwechsel, den Zellverband und/oder den internen Zellstoffwechsel insgesamt verträglich sein müssen. Andernfalls kommt nicht nur die Virusvermehrung in den Zellen zum Erliegen, sondern es wird auch das (Zell-)Leben des gesamten behandelten Organismus beeinträchtigt oder schlimmstenfalls ebenfalls beendet.

Unter dieser Prämisse beschreibt die EP 253 87 90 B1 eine "Virusinaktivierende Zusammensetzung", bestehend aus 0,02 bis 0,3 M Arginin und 0,01 bis 10 mM eines Flavonoides oder Polyphenols oder Ascorbinsäurederivates sowie 0,005 bis 5 Massen% von Acylarginin mit einer Acylkette mit 8 bis 16 Kohlenstoffatomen und davon abgeleiteten Salzen mit Säuren oder Basen. Invitro-Versuche bestätigen eine Wirkung gegen Influenza, Herpes, Rhinovirus sowie gegen Coronaviren.

Da diese Wirkstoffe körpereigenen Substanzen gleichen oder ähnlich sind, sind ihre unerwünschten Nebenwirkungen sehr gering und oft auch gar nicht feststellbar.

Eine ganz grundsätzliche Einschränkung ist, dass diese Zusammensetzung nur für eine Wirkung auf der Haut oder auf Schleimhäuten gedacht und wirksam ist. Sie muss auf diesen Körperflächen in Kontakt mit den Viren kommen. Alle Viren, die dort nicht erfasst werden, dringen weiter in den menschlichen Organismus ein, wo sie dem pauschalen Wirken dieser Zusammensetzung entzogen sind.

Eine weitere Einschränkung ist, dass die in vitro nachgewiesene Behinderung der Replikation der Viren nur dann erheblich ist, wenn ein relativ genau abgestimmtes Verhältnis der einzelnen Bestandteile eingehalten wird. Stancioiu Felician et al.: "A dissection of SARS-CoV2 with clinical implications (Review)", International Journal of molecular medicine, 2020, offenbart die Verwendung einer Kombination aus Arginin und Vitamin C zur Behandlung von Covid-19.

Auf diesem Hintergrund hat sich die Erfindung die Aufgabe gestellt, eine antivirale thrombosehemmende Zusammensetzung zu finden, die die Bildung von Thromben direkt behindert und indirekt dadurch, dass das Eindringen von Viren, insbesondere von Corona-Viren, in die menschlichen Atemwege zumindest behindert wird und die Replikation dennoch eingedrungener Viren gehemmt wird und das menschliche Immunsystem bei der Abwehr dieser Viren stimuliert und unterstützt wird, wobei kostengünstige sowie für den menschlichen Körper und den menschlichen Stoffwechsel möglichst wenig störende Wirkstoffe verwendet werden sollen.

Als Lösung dieser Aufgabe lehrt die Erfindung eine antivirale thrombosehemmende Zusammensetzung zur Verwendung bei der Behandlung von Covid-19 mit Arginin als erstem Bestandteil dadurch gekennzeichnet, dass sie als einen zweiten Bestandteil Thiocyanate, auch Rhodanid genannt, enthält, und zwar als Kaliumthiocyanat oder als ein anderes Salz oder als ein Thiocyansäureester.

Es ist das Verdienst der Erfindung, zwei für sich alleine jeweils bereits als antiviral bekannte Wirkstoffe zu wählen und miteinander zu kombinieren, so dass sich ihre Wirkung im Ergebnis nicht nur addiert, sondern gegenseitig in einem zuvor nicht ansatzweise erwartbaren Maße verstärkt. Bei Verabreichung nur eines der beiden Wirkstoffe werden Viren lediglich in ihrer Wirkung und Ausbreitung etwas gehindert und gebremst. Aber bei der erfinderischen, gemeinsamen Anwendung wird eine sehr große Anzahl von Viren daran gehindert, sich im Blutkreislauf mit den Erythrozythen zu verbinden und Thromben zu bilden, die bei den meisten, an Covid 19 erkrankten Patienten zu einer Lungenembolie und damit zum Tod durch Rechtsherzversagen geführt haben.

Weil die Zubereitung aus den beiden erfinderischen Komponenten Arginin und Rhodanid die Bindungsfähigkeit der Viren stark reduziert, schwimmen sie frei im Serum des Blutes, werden von den Scherkräften des Blutstroms beschädigt und sind für die Zellen des Immunsystems relativ sehr leicht erreichbar und zerstörbar. Nur wenige Viren gelangen noch durch das Epithel der Blutgefäße hindurch in die Zellen von Herz, Leber, Nieren und Gehirn, um sich dort zu replizieren und schlussendlich die Wirtszelle absterben zu lassen.

Damit wird der Circulus vitiosus der ansonsten expotentiell ansteigenden Anzahl von Viren unterbrochen, so dass das Immunsystem des menschlichen Körpers eine realistische Chance bekommt, die Viren nicht nur qualitativ, sondern auch quantitativ erfolgreich zu bekämpfen.

Um diese vorteilhafte Wirkung zu erläutern und plausibel zu machen, wird im Folgenden am Beispiel der Coronaviren und insbesondere des SARS-CoV-2 deren prinzipielles Eindringen und Replizieren in den menschlichen Körper erläutert sowie die Einzelwirkung der erfinderischen Komponenten Arginin und Rhodanid erläutert. Anschließend werden aktuelle Forschungsberichte zitiert, die Thromben im Blutkreislauf als eine der häufigsten Todesursachen bei Covid 19 sowie bei Krebserkrankungen herausstellen und nahelegen, dass der Eingriff in das Hämaglutinin die Bildung von Thromben weitestgehend unterbindet.

Coronaviren und insbesondere das SARS-CoV-2 zeichnet gegenüber anderen Viren ein vergleichsweise vielgliedriger Aufbau mit einer für Viren ungewöhnlich langen und daher vergleichsweise überdurchschnittlich komplexen RNA aus. Das einzelsträngige RNA-Genom der Coronaviren ist etwa 27.600 bis 31.000 Nukleotide (nt) lang, womit Coronaviren die längsten Genome aller bekannten RNA-Viren besitzen. Im Unterschied zur üblicherweise hohen Fehlerrate der RNA-Polymerase von anderen RNA-Viren, die zu einer Beschränkung der Genomlänge auf etwa 10.000 Nukleotide führt, wird bei Coronaviren eine relativ hohe genetische Stabilität (Konservierung) unter anderem durch eine 3'-5'-Exoribonuklease-Funktion des Proteins NSP-14 erreicht (M. R. Denison, R. L. Graham, E. F. Donaldson, L. D. Eckerle, R. S. Baric: Coronaviruses: an RNA proofreading machine regulates replication fidelity and diversity. In: RNA Biology. Band 8, Nr. 2, März-April 2011, S. 270-279, doi:10.4161/rna.8.2.15013, ISSN 1555-8584, PMID 21593585, PMC 3127101).

Es ist legitim, aus der gegenüber durchschnittlichen Viren dreifachen Länge des Genoms der Corona-Viren auf eine entsprechend erhöhte Vielfalt der Reaktionen jedes Virus auf seine jeweilige Umgebung sowie entsprechend vielfältigere Eingriffe und Steuerungen der Virus RNA in die DNA der Wirtszelle zu schließen.

Deshalb ist es der zentrale Gedanke der Erfindung, dem daraus resultierenden, an Varianten reichen Vorgehensweise des Virus als Gegenmaßnahme nicht nur einen einzigen Stoff entgegensetzen zu wollen, sondern ein ebenfalls variantenreiches Spektrum an verschiedenen Wirkstoffen einzusetzen, das auf die vier verschiedenen Stufen der Wirkung vor und während der Infektion abgestimmt und optimiert ist.

Die hier relevanten Stationen eines Virus sind:
1. Sein Eintritt in die Atemwege über den Mund- und Nasenraum,
2. Die noch nicht aktive Existenz im Körper,
3. Sein Eindringen in eine Wirtszelle,
4. Das Freisetzen der RNA zur Programmierung der Ribosomen der Wirtszelle für die Produktion virusspezifischer Proteine und schließlich die RNA-Replikation durch die virale RNA-Polymerase für das Zusammenlagern mit den virusspezifischen Proteinen zu neuen Viren im endoplasmatischen Retikulum.

Die Sinnfälligkeit dieser erfinderischen Idee weist der zeitliche und quantitative Ablauf einer Infektion durch SARS-CoV-2 nach. Es ist weithin bekannt, dass schon in den ersten beiden Tagen nach dem Beginn einer Infektion replizierte Viren auf andere Personen übertragen werden, obwohl der Infizierte selbst noch keine Krankheitssymptome zeigt.

Ebenfalls auffällig ist, dass viele Patienten erst dann eine positive Reaktion auf einen Virustest gezeigt haben, wenn sie über mehrere Minuten hinweg einen intensiven Kontakt mit der Atmung bereits infizierter Personen gehabt haben. Ein herausragendes Beispiel ist eine Gaststätte in Bad Ischgl, Österreich, in der sich ein infizierter Kellner durch eine dicht gedrängte Menge von Gästen einen Weg zu bahnen versuchte, indem er regelmäßig seinen Atem durch eine Trillerpfeife hindurch ausstieß. Für die Mehrzahl der Gäste wurde daraufhin eine Infektion nachgewiesen. Über Stunden hinweg wurden die Personen also immer wieder einer neuen Wolke von Viren ausgesetzt. (Spiegel, Walter Mayr, Wien, 17.3.2020, 12:23Uhr, https://www.spiegel.de/politik/ausland/coronavirus-ausbruch-in-ischgl-die-brutstaette-a-8f56e5a2-635f-473a-96e9-300b6cbf4180).

Auch andere lärmende Volksfeste oder Chorproben, bei denen sich zahlreiche Menschen und darunter wenige infizierte Individuen mehrere Stunden lang dicht aneinander drängten und durch möglichst lautes Sprechen, druckvolles Lachen bis hin zu lautem Singen regelmäßig mit SARS-Cov-2 kontaminierte Atemwolken zu ihren Nachbarn und Gegenübern sandten, haben sich als wirkungsvoller Herd für weitere Infektionen erwiesen. (Wie sich Viren beim Singen verbreiten 2.6.2020, https://futurezone.at/science/wiesich-viren-beim-singen-verbreiten/400929119, Prof. Fritz Sterz, Medizinische Universität Wien, Währinger Gürtel 18-20/6D, 1090 Wien, Österreich, Untersuchung und fotografische Dokumentation von Aerosol- und Kondenswasseremission bei Chormitgliedern)

Auffällig ist, dass medizinisch Berufstätige überdurchschnittlich oft selbst mit SARS-CoV2 infiziert werden, wenn sie mit infizierten Patienten in Kontakt kommen.

Es ist ein Verdienst der Erfindung, zu differenzieren, dass ein quantitativ geringer Kontakt mit den SARS-CoV-2-Viren ganz offensichtlich durch das körpereigene Immunsystem noch beherrscht werden kann, indem die betroffenen Wirtszellen zwar Viren replizieren, jedoch in derart geringer Anzahl, dass weder sie selbst noch das Immunsystem damit überlastet werden.

Anders ist es jedoch, wenn innerhalb kurzer Zeit eine sehr große Anzahl von Viren in einen menschlichen Organismus einströmt. Erst dann werden bei den betroffenen Patienten die Symptome einer Krankheit erkennbar. Offensichtlich direkt proportional zur Anzahl der einwirkenden Viren steigern sich die Krankheitssymptome. Verstorbene Patienten haben entweder unter einer Einschränkung ihres Immunsystems oder unter ganz regelmäßig und immer wieder auftretenden quantitativen Überlastungen durch virale Kontamination gelitten.

Daraus folgt als Kern des erfinderischen Gedankens, dass es am wirkungsvollsten ist, die Viren nicht nur qualitativ, sondern in Verbindung damit auch quantitativ zu bekämpfen, also Wirkstoffe für jede der vier klassifizierten Stufen ihres Eindringens und Wirkens in den menschlichen Organismus bereitzuhalten.

Ein direkter und frontaler, breitbandiger Angriff auf die Viren durch Desinfektionsmittel ist nur außerhalb des menschlichen Organismus sinnvoll, da diese innerhalb des Organismus nicht hinnehmbare Kollateralschäden verursachen.

Ein direktes Eintreten der Viren über unverletzte Haut, die mit viral belasteten Flächen in Kontakt gekommen ist, ist mit an Sicherheit grenzender Wahrscheinlichkeit auszuschließen. Die einzig offene Eintrittspforte bei zuvor gesunden Patienten sind seine Schleimhäute, insbesondere im Mund- und Nasenraum. Auf der Haut befindliche Viren sind nachweislich nur dadurch in den Körper eingedrungen, dass der betroffene Patient sie mit seinen eigenen Händen in Kontakt mit seinen Schleimhäuten im Bereich des Kopfes gebracht hat. Viren, die auf diese Weise oder durch die Atemluft infizierter Menschen auf die Schleimhäute gekommen sind, befinden sich noch außerhalb des Körpers. Nur in diesem Bereich besteht noch eine letzte Möglichkeit, die Viren durch einen Direktangriff soweit zu schädigen, dass sie ihren Daseinszweck, die Replikation, nicht mehr ausführen können.

In der **ersten Stufe** des Weges der Corona-Viren im menschlichen Körper, nämlich dem Eintritt in die Atemwege über den Mund- und Nasenraum können direkte Angriffe auf die Viren noch verträglich sein, jedoch nur bei sorgfältiger Beachtung der Nebenwirkungen.

Die Erfindung schlägt dazu als Wirkstoff **1,8-Cineol** vor. Bekannt ist, dass es in der menschlichen Lunge und in den Nebenhöhlen schleimlösend und bakterizid wirkt. Jetzt haben neueste Untersuchungen gezeigt, dass das 1,8-Cineol in seinem natürlichen Vorkommen als Eukalyptusöl, Pfefferminzöl oder einem anderen Minzöl in Zusammenwirkung mit den öligen Bestandteilen die Hüllproteine und die Lipidmembran des Virus deutlich verändert, wodurch der Matrixraum des Virus und die darin enthaltenen RNA-Genome soweit beschädigt werden, dass ein Durchdringen anderer Zellmembranen und ein Übertragen der RNA-Genome nicht mehr möglich ist.

Ebenfalls für die **erste Stufe** des Weges der Viren sind auch **Kupfer**sowie Zink- und Mangan Verbindungen unter gewissen Bedingungen sinnvoll. Der Zusatz von Kupfer Salzen (z.B. als CUSO₄), Zink-Salzen (z.B. Zink-Sulfat) und Mangan-Salzen (z.B. Mangansulfat) führt zur gewünschten toxischen Schädigung der Viren-Enzyme, wie z.B. der Polymerase. In der Pharmazie wird häufig Kupfersulfat-Pentahydrat (5 H₂O) verwendet, dessen antimikrobielle Eigenschaften auch gegen SARS-CoV-2 wirken. Bei der Dosierung muss jedoch Vorsicht walten, da es im Extremfall zu höchst unerwünschten Ätzungen der Schleimhäute, Erbrechen und Diarrhöe bis hin zur Hämolyse kommen kann.

Für die **zweite Stufe** der noch nicht aktiven Existenz der Viren im Körper setzt die Erfindung als alleinige oder zumindest wesentliche Bestandteile der erfinderischen Zusammensetzung eine Kombination aus Arginin und Thiocyanat ein.

L-Arginin ist eine proteinogene, semi-essentielle Aminosäure. Sie wird vom Körper selbst produziert, die Produktion reicht jedoch nicht immer aus, um den körperlichen Bedarf zu decken. Die bekannte antivirale Wirkung des L-Arginins beruht auf seiner bedeutenden Rolle im menschlichen Harnstoff- sowie im Stickstoff-Kreislauf. Arginin dient als Baustoff und damit als alleinige Vorstufe für Stickstoffmonoxid, das Blutgefäße weitet und die Entstehung von Blutgerinnseln mindert.

L-Arginin erhöht signifikant die Aktivität der Abwehrzellen innerhalb des Immunsystems.( Barbul/Efron/Lazarou/Wasserkrug: Arginine enhances wound healing and lymphocyte immune responses in humans. 1990)

Arginin verstärkt die unterdrückte Immunantwort bei schweren Verletzungen, Mangelernährung, Sepsis und nach Operationen. Bei zusätzlicher Gabe wird eine verbesserte zelluläre Immunantwort, eine Abnahme verletzungsbedingter Funktionsstörungen der T-Zellen und eine verstärkte Phagozytose beobachtet (U. Landmesser u. a.: Endothelial function: a critical determinant in atherosclerosis? In: Circulation. 109 (21 Suppl 1), 2004, S. II27-II33. PMID 15173060; PDF ; P. Fürst, H.-K. Biesalki u. a.: Ernährungsmedizin. Thieme-Verlag, Stuttgart 2004, S. 94-95)

Die Phagozytose ist die aktive Aufnahme von Partikeln bis hin zu kleineren Zellen in eine einzelne eukaryotische Zelle. Durch einen Einstülpungsvorgang, die Invagination, werden die eingeschleusten Objekte in Nahrungsvakuolen eingeschlossen, in der sie dann "verdaut", also chemisch abgebaut und damit zerstört werden. (Ronald S. Flannagan, Valentin Jaumouill'e, Sergio Grinstein: The cell biology of phagocytosis. In: Annual Review of Pathology: Mechanisms of Disease. Band 7, 2012, S. 61-98 doi:10.1146 /annurev-pathol-011811-132445.)

Dass der Mensch innerhalb seines Harnstoffzyklus Arginin selbst synthetisieren kann, weist die Verträglichkeit von Arginin nach. Wenn die entstehenden Mengen nicht ausreichen, um den Bedarf bei Stress und Krankheiten wie z. B. Arteriosklerose, Bluthochdruck, Gefäßerkrankungen oder nach Unfällen durch die körpereigene Produktion vollständig zu decken, ist die Gabe von Arginin hilfreich. (A. Ströhle, A. Hahn: Arginin bei Atherosklerose: Diätetische Maßnahmen bei Atherosklerose - Stellenwert von L-Arginin. In: Deutsche Apotheker Zeitung. Teil 1: Band 20, 2012, S. 97-102 (online, freier Volltext) und Teil 2: Band 21, S. 74-83 (online, freier Volltext). Sowie I. Seljeflot I., B. B. Nilsson, A. S. Westheim, V. Bratseth, H. Arnesen: The L-arginine-asymmetric dimethylarginine ratio is strongly related to the severity of chronic heart failure. No effects of exercise training. In: J. Cardiac. Fail. Band 17, 2011, S. 135-142). Das beweist, dass die erfinderische Beimischung von Arginin als körpereigenem Stoff aufgabengemäß praktisch keine unerwünschten und unverhältnismäßigen Nebenwirkungen generiert.

Für die große Bedeutung von **Thiocyanat** im Zellstoffwechsel dürfte die exogene und endogene Präsenz während der Entwicklung des Lebens Voraussetzung gewesen sein. (W. Weuffen, A. Kramer, H. Ambrosius, V. Adrian, H. Below, W. D. Jülich, S. Koch, B. Thürkow, F. Verbeek: Zur Bedeutung des endogenen Wirkstoffs und Umweltfaktors Thiopcyanat für die unspezifische und spezifische Resistenz aus hygienischer Sicht. In: Zentralblatt für Hygiene und Umweltmedizin. 189, 1990, S. 473-510)

Die durch Oxidation entstehenden Hypothiocyanite sind antimikrobiell hochwirksam und essenziell für die mikrobielle Abwehr in der Mundhöhle und in den Atemwegen (R. Ihalin, V. Loimaranta, J. Tenovuo: Origin, structure, and biological activities of peroxidases in human saliva. In: Arch Biochem Biophys. 445(2), 2006, S. 261-268.) Das Ion des Thiocyanats, SCN- koordiniert als Ligand in Komplexen sowohl über das Stickstoff- als auch über das Schwefelatom an das Zentralatom. Seine herausragende biologische Aktivität ergibt sich durch die vielfältigen Möglichkeiten seiner 16 Elektronen, sich anzuordnen und zu verteilen. Neben ionischen Wechselwirkungen können sie koordinative Bindungen über N-S-Ligatoratome in Form von ein- bis fünfzähnigen Verknüpfungen sowie eine kovalente oder koordinative Fixierung zu Rezeptoren und Bindungspartnern eingehen: (Siegfried Hauptmann: Organische Chemie. 2., durchgesehene Auflage. Deutscher Verlag für Grundstoffindustrie, Leipzig 1985, ISBN 3-342-00280-8, S. 470.)

Thiocyanat stimuliert die Phagozytose und damit auch die Wirkung der Immunzellen. Die erfinderische Zusammensetzung von Thiocyanat mit 1,8-Cineol führt zur expotensiellen Aktivierung von Hypothiocyanite. Dies zeigt sich dadurch, dass in wenigen Sekunden eine Zerstörung des Virus eintrat. Das Therapeutium wirkt antiphlogistisch und protektiv besonders bei infektiöser Belastung. Die antiinfektiöse Schutzwirkung beruht sowohl auf der Förderung der Kolonisationsresistenz als auch indirekt durch Bildung von Hypothiocyanit.

Für Benzylisothiocyanat und Allylisothiocyanat ist eine hemmende Wirkung auf Viren nachgewiesen. Sie sind Bestandteile des Senföls, das gegen Atemwegsinfektionen durch Viren eingesetzt wird. Für Senföle mit einem Gehalt an Benzylisothiocyanat und Allylisothiocyanat zeigten In-vitro-Untersuchungen durch Professor Stephan Pleschka vom Institut für Medizinische Virologie der Universität Gießen, dass diese die Vermehrung von Viren in mit dem Influenza-A-Virus H1N1 infizierten Lungenepithelzellen um bis zu 90 % vermindern können. Pleschka und seine Mitarbeiter gehen davon aus, dass die Senföle nicht direkt das Virus angreifen, sondern die Interaktion zwischen den Wirtszellen und dem Virus zum Nachteil des Virus stören. Es ist evident, dass ein solches Wirkprinzip besser vor viralen Resistenzentwicklungen schützt als ein direkter antiviraler Angriff. (Werner Stingl: Influenza-Viren mit Phytotherapie bekämpfen. In: Ärzte Zeitung. 16. Dezember 2010.)

Ebenfalls in der zweiten Stufe eines Virus im menschlichen Körper, nämlich dem noch frei beweglich vagabundierenden Status ist das wesentliche Merkmal der Erfindung die ganz überraschend intinsive, gegenseitige Verstärkung der antiviralen Wirkung bei der gemeinsamen Verabreichung von Arginin und Rhodanid, heute auch Isothyocynat genannt. Insbesondere wirken sie der in Intensiv-Stationen vielfältig beobachteten Bildung von Thromben im Blutkreislauf von Covid-19-Patienten entgegen.

Im April 2020 veröffentlichte die niederländischen Zeitschrift Thrombosis Research (2020: DOI: 10.1016/j.thromres.2020.04.028) eine Studie, gemäß der es bei fast jedem dritten von 184 untersuchten Coronapatienten thrombotische Komplikationen gab. Stavros Konstantinides, Direktor des Zentrums für Thrombose und Hämastose am Universitätsklinikum Mainz schätzt, dass von den Covid-19-Patienten, die stationär behandelt werden mussten, etwa jeder vierte eine "mehr oder weniger schwere Thrombose und Lungenembolie entwickelt". Konstantinides geht davon aus, dass SARS-Cov-2 Viren an Rezeptoren der Endothelzellen in Blutgefäßen andocken. "Auf diese Weise könne SARS-CoV-2 kleinere und auch größere Blutgefäße befallen, so dass Thrombosen entstünden." (Tödliche Blutgerinnsel bei Covid-19-Patienten, von Ilona Kriesl, Redakteurin für Wissenschaft und Gesundheit, Stern, 13.5.2020)

Prof. Klaus Püschel, Direktor des Instituts für Rechtsmedizin des Universitätsklinikums Hamburg-Eppendorf, UKE, verweist darauf, dass bei der Therapie von Covid-19-Patienten zunehmend "auf das Blutgerinnungssystem und die Bildung von Thromben gezielt eingegangen wird. Also eine intensivere Vorsorge dafür getroffen wird, dass Thrombosen und Embolien nicht entstehen".

Prof. Stefan Kluge, Direktor der Klinik für Intensivmedizin des UKE, stellt fest, dass im klinischen Alltag COVID-19 in ungewöhnlich vielen Krankheitsfällen zu Thrombosen sowie Lungenembolien geführt hat. (NDR Presseportal 7.5.2020) .

Die Ärztin Shari Brosnahan vom Universitätskrankenhaus Langone in New York berichtet von durchgeführten oder drohenden Amputationen der Beine und der Finger bei 40-jährigen Patienten auf der Intensivstation. Autopsien zeigten sehr zahlreiche, winzige Gerinnsel in den Lungen Verstorbener. Cecilia Mirant-Borde, Intensivmedizinerin am Militärkrankenhaus Manhattan, NewYork, berichtet, dass künstliche Beatmung vielen Patienten mit Sauerstoffmangel im Blut nicht hilft. Sie geht davon aus, dass Mikro-Gerinsel in der Lunge die Blutzirkulation und damit die Sauerstoffversorgung blockieren. (29. April 2020, https://www. aerzteblatt.de/ nachrichten/112427/Raetselhafte-Blutgerinnsel-bei-COVID-19-Patienten)

Dominic Wichman vom Universitätsklinikum Hamburg Eppendorf, UKE, berichtet von der Obduktion von zwölf nachweislich mit dem Coronavirus infizierten und daran verstorbenen Patienten. Sieben Patienten hatten eine tiefe Venenthrombose gebildet, obwohl vor ihrem Tod kein Verdacht auf eine Thrombose bestand. Bei vier Patienten wanderte ein gelöstes Gerinnsel in die Lunge, verstopfte ein Gefäß und löste eine Lungenembolie aus, die dann direkte Todesursache war. Die Verstorbenen waren zwischen 52 und 87 Jahren alt, überwiegend männlich und vorerkrankt. 6 Patienten litten an koronaren Herzkrankungen und Asthma, drei an COPD (Raucherlunge).

In den Lungen konnten hohe Konzentrationen von Sars-CoV-2 nachgewiesen werden. Bei 6 Patienten fand sich der Virus im Blut, in 5 Fällen in Leber, Nieren oder Herz. Dominic Wichmann und Kollegen schließen daraus, dass sich das Virus über die Blutgefäße im Körper verbreitet und Einfluss auf die Blutgerinnung nimmt. (Autopsy Findings and Venous Thromboembolism in Patients With COVID-19, A Prospective Cohort Study, Dominic Wichmann, MD Jan-Peter Sperhake, MD Marc Lütgehetmann, et al., Annals of Internal Medicine, 6.Mai 2020)

Das Risiko einer Thrombose durch COVID-19 sei so hoch, dass Patienten "möglicherweise prophylaktisch Blutverdünner verabreicht werden sollten", resümiert Behnood Bikdeli, Irving Medical Center, New York (Journal of The American College of Cardiology (2020; DOI: 10.1016/j.jacc.2020.04.031). Shari Brosnahan bestätigt, dass bei einigen Patienten Heparin wirkte, aber bei anderen nicht. Es gibt also verschiedene Zusammensetzungen bei den Thromben. Der Verursacher der Thromben ist das Virus selbst, was auch von anderen Viren bekannt ist. Dazu trägt bei, dass das Virus auch das Endothel der Blutgefäße infiziert (The Lancet 2020; doi: 10.1016/ S0140-6736(20)30937 -5).

Der vielschichtige, von etlichen Faktoren beeinflusste und auf zahlreichen chemischen Zusammensetzungen basierende Prozess der Thrombenbildung oder Agglutinierung der Thrombozyten wird als Hämagglutination bezeichnet. Je nach wesentlichen Bestandteilen der Thromben wird unterschieden zwischen den weißer Thromben (Abscheidungsthrombus) mit hohem Anteil an Fibrin, den roten Thromben (Gerinnungsthrombus) mit einem hohen Anteil an Blut und dem Plättchenthrombus, der vorwiegend aus Thrombozyten besteht.

Bei allen Arten der Thromben sind an der Hämagglutination die Hämagglutinine wesentlich beteiligt, die als Oberflächenantigene bei einigen Viren vorkommen. Bekannt ist zum Beispiel das Influenza-Hämagglutinin oder das Masern-Hämagglutinin. Bei der Infektion einer Wirtszelle vermitteln Hämagglutinine die Anheftung des Virus an die Zellen und erleichtern sein Eindringen in die Wirtszelle, indem sich das Hämagglutininmolekül an große, sialinsäuretragende Glykoproteine auf der Zelloberfläche anbindet. Die Sialinsäuren fungieren als Rezeptoren. Das angeheftete Virus wird dann per Endozytose in die Zelle aufgenommen ( Flexikon 1.6.2020, Dr. Frank Antwerpes, DocCheck Community GmbH, Vogelsanger Straße 66, 50823 Köln).

Eine hier besonders wichtige Sialinsäure ist die Neuraminsäure auf der Oberfläche einer Wirtszelle, die der Rezeptor für die homotrimeren Membranproteine des Hämaglutinins (HA) ist. Es steht als etwa 10 bis 14 Nanometer langes Peplomer aus der Virushülle wie die Zacken einer Krone heraus und hat durch dieses äußere Erscheinungsbild den Corona-Viren ihren Namen verliehen. Das HA ist ein Trimer aus drei identischen Einheiten, die nach ihrer proteolytischen Spaltung aus zwei Untereinheiten bestehen, die durch eine Disulfidbrücke miteinander verbunden sind.

Diese Brücke ist mit sehr hoher Wahrscheinlichkeit ein wesentlicher Angriffspunkt für die erfinderische Zusammensetzung. Elektronenmikroskopische Untersuchungen haben das starke Dipolmoment der erfinderischen Molekülstruktur von Arginin-Rhodanid aufgezeigt. Da Schwefel-Schwefel-Verbindungen nicht besonders stabil sind, führt dieses hohe Dipolmoment zur Aufspaltung der Schwefel-Schwefel-Verbindungen und damit zu einer so tiefgreifenden Veränderung des Hämagglutinins des Virus, dass dessen Eindringen in eine Wirtszelle kaum mehr möglich ist.

Es ist bekannt, aber bisher therapeutisch nur peripher verfolgt, dass Tiefe Venenthrombose (TVT) und Lungenembolie (LE), zusammengefasst unter dem Begriff der venösen Thromboembolie(n) (VTEs), häufig bei Tumorpatienten auftreten (Thrombose und Krebs: Prophylaxe und Therapie, Dtsch Arztebl 2017; 114(48): [4]; DOI: 10.3238/PersOnko/2017.12.01.01,Habbel, Piet; Riess, Hanno). Sie verschlechtern die Prognose der Patienten und sind eine wesentliche Ursache für die Mortalität (Lubberts B, Paulino Pereira NR, Kabrhel C, et al.: What is the effect of venous thromboembolism and related complications on patient reported health-related quality of life? A meta-analysis. Thromb Haemost 2016; 116: 417-31 CrossRef MEDLINE sowie Khorana AA, Francis CW, Culakova E, et al.: Thromboembolism is a leading cause of death in cancer patients receiving outpatient chemotherapy. J Thromb Haemost 2007; 5: 632-4 CrossRef MEDLINE).

Es ist ein weiterer wichtiger Effekt der erfinderischen Kombination von Arginin und Rhodanid, dass die Formation des viralen und tumorösen Hämagglutinins durch die Freisetzung von Stickstoffmonoxid (NO) weiter verändert wird, so dass eine zusätzliche Modifikation das Andocken an Erythrozyten, Thrombozyten oder Endothelzellen erschwert und damit die Thrombenbildung spürbar behindert. Das ist plausibel, weil - wie bereits erwähnt - Arginin der Baustoff und die alleinige Vorstufe für NO ist, das bekanntlich Blutgefäße weitet und die Entstehung von Blutgerinnseln mindert.

In der **zweiten Stufe** der Wirkung frei beweglicher Viren oder Tumorzellen kann deren Anregung zur Bildung von Thromben auch dadurch verstärkt entgegengetreten werden, dass die erfinderischen Zusammensetzung zusätzlich **Dimethylsulfoxid** (DMSO) enthält. Es ist bekannt, dass DMSO zum schnellen Abschwellen von Blutergüssen beiträgt (Arnd Krüger: DMSO. In: Leistungssport. 43, 3, 2013, S. 28.)

Als **dritte Stufe** eines Virus im menschlichen Körper bezeichnet die Erfindung dessen Eintreten in eine Wirtszelle. Dem kann die Erweiterung der erfinderischen Zusammensetzung um Methylsulfonylmethan (MSM) entgegentreten: MSM unterdrückt bekanntermaßen allergische Reaktionen, in dem es Rezeptoren besetzt, an die Allergene andocken. Da an diese Rezeptoren auch Viren oder Tumorzellen andocken oder versuchen anzudocken, ist die Blockade dieser Rezeptoren ein weiterer Teilschritt beim erfolgreichen Zurückdrängen der massenhaften Ausbreitung von Viren oder Tumorzellen.

Ebenfalls in der **dritten Stufe** einer Vireneinwirkung könnten, wie im Folgenden plausibilisiert wird, **ACE-Hemmer** wirksam werden. Für das Eindringen der SARS-CoV-2- und anderer humaner Coronaviren in eine Wirtszelle haben sich insbesondere die Atemwege und die Atmungsorgane als vergleichsweise gängige Pforte erwiesen. Dort verursachen die Viren Infektionen der oberen Luftwege bis hin zu schwersten Lungenentzündungen oder gar Lungenversagen.

A.S. Fauci, C.I. Paules und H.D. Marston, National Institute of Allergy and Infectious Diseases, Bethesda, USA, schreiben, dass Corona-Viren diejenigen Moleküle auf Zelloberflächen, die das Virus zum Andocken braucht, bei Menschen vor allem in tieferen Regionen der Atemwege vorfinden. (23.Januar 2020, Coronavirus Infections, More Than Just the Common Cold, Catharine I. Paules, MD1; Hilary D. Marston, MD, MPH2; Anthony S. Fauci, MD2, JAMA. 2020;323(8):707-708. oi:10.1001/jama.2020.0757)

Mit Versuchsreihen an der University of Texas in Austin zeigten Daniel Wrapp und Jason McLellan am 20.2.2020, dass das Andocken von SARS-CoV-2 an die Wirtszelle mittels der morphologisch keulenförmigen Spikes oder Peplomere auf der Oberfläche ähnlich abläuft, wie bei den eng verwandten SARS und MERS-Erregern: Das Glycoprotein auf den Spikes klappt von einer inaktiven in eine bindefähige, aktive Konformation auf. SARS-CoV-2 ist jedoch offenbar besser an die menschliche Zelle angepasst als seine bereits bekannten Verwandten. Die ACE2-Rezeptoren der Wirtszellen binden sich mit zehn- bis 20-fach höherer Affinität an die Spike-Bindestelle des SARS-CoV-2 als bei dem bekannten SARS-CoV, was erklärt, warum sich dieses Virus so leicht von Mensch zu Mensch verbreitet.

SARS-CoV-2 dockt über die Bindung des viralen Glykoproteins S an den ACE2-Rezeptor der Wirtszelle an. Der ACE2-Rezeptor der Wirtszellen könnte deshalb ein möglicher Ansatzpunkt für eine Therapie sein (Qiu, Y.; Zhao, Y.; Wang, Q.; Li, J.; ZHou, Z.; Liao, C.; Ge, X. Predicting the Angiotensin Converting Enzyme 2 (ACE2) Utilizing Capability as the Receptor of SARS-CoV-2. Preprints 2020, 2020030091 (doi: 10.20944/preprints202003.0091.v1).

Für den Zelleintritt aller Coronaviridae sind neben den Enzymen Trypsin und Furin auch die Proprotein-Konvertasen wie Cathepsine, Elastasen und insbesondere Transmembranserin-proteasen, TMPRSS wichtig. Die im Atemtrakt vermehrt vorhandenen und an Zelloberflächen exprimierten Proteasen TMPRSS2 und TMPRSS11a begünstigen den Eintritt von SARS-CoV-1-Viren. Für die TMPRSS-Protease TMPRSS11d - auch als human airway trypsin-like protease (HAT) bekannt - wurde eine proteolytische Aktivierung des S-Proteins von SARS-CoV-1 nachgewiesen. TMPRSS2 bildet wiederum mit dem ACE2-Rezeptor einen Komplex, und ermöglicht dadurch ein effizientes Eindringen des Virus direkt an der Zelloberfläche. (Jean Kaoru Millet, Gary R. Whittaker: Host cell proteases: critical determinants of coronavirus tropism and pathogenesis. In: Virus Res. 16. April 2015, doi:10.1016/j.virusres.2014.11.021 (englisch). sowie A. Shulla, T. Heald-Sargent, G. Subramanya, J. Zhao, S. Perlman, T. Gallagher: A transmembrane serine protease is linked to the severe acute respiratory syndrome coronavirus receptor and activates virus entry. In: Journal of Virology. Band 85, Nummer 2, Januar 2011, S. 873-882, doi:10.1128/JVI.02062-10, PMID 21068237, PMC 3020023 sowie Shirato K, Kanou K, Kawase M, Matsuyama S.: "Clinical Isolates of Human Coronavirus 229E Bypass the Endosome for Cell Entry." In: J Virol. Dec 2016, doi:10.1128/JVI.01387-16, PMID 27733646)

Das Enzym TMPRSS2 der Wirtszelle aktiviert also die weiteren Schritte des Infektionsvorganges, die zum Eindringen des Erregers in die Wirtszelle notwendig sind. Damit ist TMPRSS2 ein weiterer potentieller Ansatzpunkt für ein wirksames Medikament (Hoffmann et al., SARS-CoV-2 Cell Entry Depends on ACE2 and TMPRSS2 and Is Blocked by a ClinicallyProven Protease Inhibitor, Cell (2020), https://doi.org/10.1016/j.cell.2020.02.052).

SARS-CoV-2 dringt hauptsächlich in Alveolarepithelzellen ein, was zu respiratorischen Symptomen führt. Diese Symptome sind bei Patienten mit Herz-Kreislauf-Erkrankung schwerwiegender, aufgrund einer vermutlich erhöhten ACE2-Sekretion im Vergleich zu gesunden Personen. Die sog. konterregulatorische Achse, counter-regulatory axis, des Renin-Angiotensin-Aldosteron-Systems (RAAS), die vom Enzym ACE2 und seinen Produkten Angiotensin-(1-9) und Angiotensin-(1-7) ausgeht, nimmt eine schützende Rolle bei Herz-Kreislauf-Erkrankungen ein. ACE-Hemmer und Angiotensin-Rezeptorblocker (ARB) reduzieren das Angiotensin-II-Niveau und aktivieren dadurch die konter regulatorische Achse. (Ying-Ying Zheng, Yi-Tong Ma, Jin-Ying Zhang, Xiang Xie: Reply to: 'Interaction between RAAS inhibitors and ACE2 in the context of COVID-19'. In: Nature Reviews Cardiology. 30. März 2020, doi:10.1038/s41569-020-0369-9.)

Zum Zeitpunk der Anmeldung dieser Erfindung wird COVID-19 und Behandlung mit Hemmstoffen des Renin-Angiotensin-Systems kontrovers diskutiert. Da eine Infektion mit SARS-CoV2 Angiotensin II erhöht, gibt es auch Überlegungen, dass die Gabe eines Sartans oder ACE-Hemmers die Anfälligkeit für eine Lungenschädigung senken könnte. (Prof.Dr.Michael Böhm, Homburg,Saar, Pressesprecher der Deutschen Gesellschaft für Kardiologie - Herz- und Kreislaufforschung e.V. Grafenberger Allee 100 40237 Düssel-dorf).Vorbehaltlich weiterer diesbezüglicher Erkenntnisse schlägt die Erfindung deshalb Sartan oder einen **ACE-Hemmer,** als weiteren Bestandteil der antiviralen Zusammensetzung vor.

Für die **vierte Stufe** des Wirkens eines Corona-Virus, nämlich das Freisetzen und Replizieren der RNA, offenbart die vorliegende Anmeldung in der Planung der Verabreichung der erfinderischen Zusammensetzung als wirkungsvolle Ergänzung die Umstellung auf **ketogene Ernährung,** um einen anaeroben Stoffwechsel in den befallenen Zellen zu vermeiden

Nach dem Eindringen in die Wirtszelle programmieren der SARS-CoV-2 Virus ebenso wie auch andere Viren die DNA der Wirtszelle zur viralen RNA Neuproduktion in mehreren Schritten. Dabei ist sowohl für die Synthese der RNA als auch der Membran als "Baumaterial" in erhöhten Mengen Stickstoff erforderlich. Dieses Volumen ist nur dann verfügbar, wenn die Wirtszelle ihren aeroben Stoffwechsel einstellt. Der dadurch ausgelöste, intrazelluläre Abbau erzeugt genügend Abfallprodukte, aus denen der erforderliche Stickstoff entnommen werden kann. Der daraufhin einsetzende anaerobe Stoffwechsel bewirkt ein saures Zellmilieu, was für die Replikation der Viren ebenfalls erforderlich ist.

Als Gegenmaßnahme muss der aerobe Stoffwechsel durch ausreichende Sauerstoffversorgung aufrecht erhalten und die Übersäuerung des Organismus verhindert werden.

Deshalb muss die erfinderische Zusammensetzung in ihrer Wirkung dadurch unterstützt werden, dass sich der Organismus auf einen aeroben Stoffwechsel stützt. Die wirksamste Maßnahme dafür ist es, auf eine ketogene Ernährung umzustellen. Das bedeutet, dass Kohlehydrate möglichst wenig und Glucose-Lösungen gar nicht verwendet werden dürfen.

Im Folgenden sollen weitere Einzelheiten und Merkmale der Erfindung anhand konkreter Beispiele aus der Praxis des Anmeldenden näher erläutert werden. Diese sollen die Erfindung jedoch nicht einschränken, sondern nur erläutern.

Bisher über 50 Patienten, deren Krankheitsbild eine massive Bildung von Thromben mit der Folge von Embolien erwarten ließ, wurden erfolgreich mit einem täglichen Spray in Nase oder Rachen behandelt.
1 Spray Stoß (0,1ml) in die Nase enthält
2,5 mg Arginin-Rhodanid
2,5 mg Methylsulfonylmethan
2,5 mg Dimethylsulfoxid
0,05 mg Kupfersulfat
0,05 mg Mangan

Das Rachenspray ist 10-fach stärker konzentriert.

Da die chemische Wirkung der erfinderischen Zusammensatzung im Hämagglutinin der Viren natürlich nicht ohne weiteres direkt sichtbar gemacht werden kann, wurde als indirekte Messgröße bei allen behandelten Patienten der Gehalt an Fibrinogen und D-Dimere im Blut regelmäßig überprüft.

Das Fibrinogen, das auch als das "Substrat der Gerinnung" bezeichnet wird, ist ein Glykoprotein, das bei der Blutgerinnung in Fibrin umgewandelt wird, welch letzteres zusammen mit zellulären Elementen des Blutes, namentlich den Thrombozyten, den Thrombus bildet. Fibrinogen setzt sich aus den drei Untereinheiten (α, β, γ) zusammen, die durch Disulfidbrücken miteinander verbunden sind. Fibrinogen kann durch einen kaskadenartigen, mehrfach rückgekoppelten und an der Oberfläche der aktivierten Thrombozyten ablaufenden Prozess der Aktivierung der im Plasma zirkulierenden prokoagulatorischen Faktoren das zentrale Enzym Thrombin bilden. Es werden Fibrinmonomere freigesetzt, die spontan zum wasserunlöslichen Fibrin-Blutgerinnsel polymerisieren. Durch weitere kovalente Quervernetzung mittels des im Blut vorhandenen Faktors XIIIa entsteht der stabile Thrombus. Der Normalwert für Fibrinogen liegt zwischen 1,8 und 3,5 Gramm pro Liter. Die im Folgenden vorgestellten Patienten hatten anfangs 3,6 bis 5,2 g/l.

Da eine Thrombose wie z.B. eine Lungenembolie oftmals klinisch inapparent verlaufen kann, war ein weiteres sensitives Testverfahren zum Nachweis bzw. Ausschluss notwendig. Ein solches Ausschlussverfahren sind die sog. D-Dimere. D-Dimere sind Fibrinabbauprodukte, die bei einer Gerinnungsaktivierung durch die parallel auch immer stattfindende Fibrinolyse entstehen. Ein negativer D-Dimer-Test schließt eine Thrombose mit hoher Wahrscheinlichkeit aus (https://flexikon.doccheck.com/de/Thrombose#Ursachen)

Der Normalwert für D-Dimere sollte kleiner als 0,5 mg/l Plasma sein. Die im Folgenden vorgestellten Patienten hatten anfangs 1,25 bis 5,22 mg D-Dimere pro Liter Plasma. Dieser Messwert war mehrfach Anlass, nach Thromben zu suchen.
**1. Behandlungsbeispiel:** Patient mit stark entzündetem Rachen und deutlichen, sich verschlimmernden Atembeschwerden in Erwartung der Einweisung zur klinischen Intensivbehandlung und Beatmung. Patient im Nachhinein Covid19-positiv getestet nach Infektion durch regelwidrige Hochzeitsfeier mit allen Personen ohne Mundschutz. Sofortige Verabreichung der erfinderischen Zusammensetzung als 10fach höher dosiertes Rachenspray. Schon nach 10 Minuten deutliche Verbesserung der Atembeschwerden. 3 x tägliche Verabreichung des Rachensprays für 5 Tage. Keine Klinikeinweisung, sondern nur häusliche Quarantäne erforderlich.
**2. Behandlungsbeispiel** ist ein Covid 19 Patient, Lebensalter 39, keine Vorerkrankungen, Handballspieler im Turniersport. Seine massiven Sprachstörungen sind eine typische Beeinträchtigung durch SARS-CoV-2. Nach Behandlung mit 3 x 5 Spraystößen täglich in den Rachen über 5 Tage, hatte sich bereits nach zwei Tagen die Sprachhemmung (am Telefon) aufgehoben. Mit sehr hoher Wahrscheinlichkeit verursachten - wie zuvor erläutert - kleinste Thromben in den Kapillargefäßen des Blutkreislaufs im Gehirn das Aussetzen der betroffenen Hirnregionen. Auch die Neverschädigungen des Nervus recurens wurde ebenfalls aufgehoben.

Die zeitliche Koinzidenz ist als Wirkungsnachweis zu werten.

**Weitere Patienten** mit dem Risiko einer massiven und durch Lungenembolie sogar letalen **Thrombenbildung** waren Krebspatienten mit Metastasen. Metastasierende Tumorzellen haben ein ähnliches Verhalten wie Viren. Durch ihre Entartung ist das Wachstum der Zelle so dramatisch verstärkt, dass es alle anderen Funktionen der Zelle überlagert oder sogar ausschaltet. Das dkfz. Deutsche Krebsforschungszentrum in der Helmholtz-Gemeinschaft beschreibt das mit Stand vom 6.9.2018 wie folgt: Metastasierende Tumorzellen haben zu Beginn ihrer Wanderung die starke Verbindung zu ihren Nachbarzellen gelöst. Weil Blutgefäße normalerweise nicht besonders gut von anderen Zellen durchdrungen werden können, beeinflussen die Tumorzellen das Epithel derart, dass es durchlässiger wird. Wenn Tumorzellen von Zellen des Immunsystems identifiziert und angegriffen werden, und einige diesen Angriff überstanden haben, dann kann es ihnen gelingen, eine Gefäßwand zu durchdringen, indem sie den Immunzellen folgen. Innerhalb der Gefäße sind sie durch den Blutstrom mechanischen Scherkräften ausgesetzt. Und sie können noch besser als zuvor vom Immunsystem des Patienten identifiziert werden. Tumorzellen interagieren deshalb zum Beispiel mit Blutplättchen: Diese schützen sie beim Transport vor Schäden und der Erkennung durch Immunzellen (https://www.krebsinformationsdienst.de/tumorarten/grundlagen/metastasenbildung. php).

In Bezug auf die Bildung von Tumoren ist ein gleiches Verhalten von Sars-CoV-2 Viren bekannt. Deshalb sind die Behandlungsergebnisse der Patienten mit metastasierenden Krebserkrankungen ein relevanter Nachweis für die Wirkung der erfinderischen Zusammensetzung auf Thromben bildende Viren wie SARS-CoV-2

**3.** Behandlungsbeispiel - nicht unter dem Wortlaut der Ansprüche: Patientenalter 76 zu Behandlungsbeginn, metastasierendes Prostatakarzinom; Polymorbidität. Weitere Bestrahlung, Operation oder Chemotherapie nicht mehr möglich, da Multiorganversagen drohte. Nur noch Palliativbehandlung geplant. Seit 8 Jahren erfinderische Zusammensetzung täglich in Kapselform, gleich dem Rachenspray. Seitdem wieder alleine Gehfähig. Reduktion der rheumatoiden Schmerzen auf etwa ein Zehntel der ursprünglichen Schmerzempfindung. Verdreifachung der subjektiv bewerteten Lebensqualität.

**4.** Behandlungsbeispiel - nicht unter dem Wortlaut der Ansprüche: Patientenalter 53 zu Behandlungsbeginn, inoperables metastasierendes sarkomatoides Nierenzellkarzinom von über 10 cm Größe, Lungenmetastasen, Peritoinalkarzinose, Perianalvenenthrombose, Ileus, Totalresektion des Omentum majus.

Seit 5 Jahren tägliche Verabreichung der erfinderischen Zusammensetzung. Monatliche Kontrollen zeigen eine Rückbildung der Symptome. Das zweimal jährlich durchgeführte PET-CT lässt keine Thrombosen oder Embolien mehr erkennen. Rückbildung der Lungenmetastasen auf passive Bindegewebsreste erkennbar nach 1 Jahr Gabe der erfinderischen Zusammensetzung. Im April 2020 keine positiven Tumormarker, Nierenwerte und Leberwerte sind im Normalbereich.

**5.** Behandlungsbeispiel - nicht unter dem Wortlaut der Ansprüche: Patientinnenalter 65 Jahre bei Behandlungsbeginn. Deutliche genetische Vorbelastung mit Mama- und Ovarial-Karzinomen sowie Leber- und Lungen- Metastasen. Behandlungsbeginn vor 8 Jahren wg. Thrombose. Dabei Mama-Karzinom diagnostiziert. Nach radikaler Mastektomie erneute Thrombose. Vor 5 Jahren Diagnose von Eierstockkrebs. Beendigung von Chemotherapie und Bestrahlung. Stattdessen seitdem kontinuierlich fortgesetzte, tägliche Gabe der erfinderischen Zusammensetzung.

Vor der ersten Behandlung praxisinterner Tumormarker K-Toxin über 250IE. Am 20.4.2020 im Normbereich bei 25 IE. Kein Thrombus, kein Tumor mehr feststellbar.

**6.** Behandlungsbeispiel - nicht unter dem Wortlaut der Ansprüche: Patientinnenalter 57 bei Behandlungsbeginn vor 9 Jahren wg. Mama-Karzinom rechts. Vor 5 Jahren Ovarialkarzinom mit Thrombose und Lungenembolie, Peritonialkarzinose, sowie Metastasen in Leber und Lungen. Beendigung von Chemotherapie und Bestrahlung. Stattdessen seitdem kontinuierlich fortgesetzte, tägliche Gabe der erfinderischen Zusammensetzung.

Im April 2020 liegen die Tumormarker CEA und CA15-3 sowie der praxisinterne K-Toxin-Wert im Normalbereich.

Auch wenn tumorassoziierte Thromboembolien (TAT) bei Krebserkrankungen vermehrt auftreten, so gilt das möglicherweise nicht für alle Tumorarten gleichermaßen: Vor allem hämatologische Malignome, Bronchialkarzinome und gastro-intestinale Tumoren führen teils zu einer mehr als zwanzigmal so hohen Inzidenz, schreiben Dr. Caio J. Fernandes- von der Universitätsklinik Säo Paulo und Kollegen. Weniger hoch, aber wegen der Häufigkeit der Erkrankungen mindestens ebenso relevant ist das Risiko bei Mamma- und Prostatakarzinomen.

**7.** Behandlungsbeispiel - nicht unter dem Wortlaut der Ansprüche: 42 weitere Patientinnen mit Mamma- und/oder Ovarial-Karzionom unter genetischer Vorbelastung wie z.B. BRCA-Mutation-1. In einigen Fällen zusätzlich Metastasen in Leber und Peritoneum. In allen Fällen tägliche Gabe der erfinderischen Zusammensetzung als Therapie. Bei allen Patientinnen reduzierten sich die Werte für Fibrinogen und D-Dimere auf den Normalbereich. Keine Thrombenbildung mehr diagnostizierbar. Kein Rezidiv, sondern kontinuierliche Rückbildung der Karzinome. Kein Todesfall durch Thromben und/oder Tumore nach Behandlungsbeginn.

In der **Gesamtschau** passen sich alle zuvor genannten Einzelfälle einer Thrombus-Bildung durch das Eindringen und aggressive Replizieren vagabundierender Viren oder Zellen an die Altersverteilung der an Covid-19 erkrankten Patienten an: Mit zunehmendem Lebensalter nehmen die Krankheitssymptome überproportional zu. Kinder und Jugendliche tragen zwar oft die Infektion mit SARS-CoV-2 weiter, sind aber selbst fast immer symptomfrei. Ausnahmefälle haben stets unter Vorbelastungen wie z.B. Diabetes, Übergewicht oder Herzschwäche gelitten.

Dieses Bild entspricht dem typischen Spiegel von Thiocyanat, auch SCN oder Rhodanid genannt. Bereits der Speichel Neugeborener enthält SCN. Der normale Thiocyanat-Serumspiegel in der Körperflüssigkeit beträgt durchschnittlich nur 2-6mg/l. Bei Körperflüssigkeiten, über die potentielle Krankheitserreger in den Körper gelangen können, wie Speichel in der Mundhöhle, Nasensekret, Tränenflüssigkeit, Magensaft und Urin kann die Konzentration im Extremfall auf 8 bis 60 mg/l ansteigen. Bei der Belastung des Körpers durch Infektionen oder Tumorerkrankung erhöht sich der Thiocyanatspiegel innerhalb kürzester Zeit auf 10-20 mg, in besonderen Situationen sogar auf 25 mg pro Liter Serum. Diese körpereigene Mobilisierung von Thiocyanat ist Ausdruck von diversen Regulationsprozessen, da in der Initialphase akuter Erkrankungen bzw. bei akuten Schüben chronischer Erkrankungen ein erhöhter Bedarf an Thiocyanat- besteht.

Etwa 60-70% der im Organismus vorhandenen Thiocyanat-Menge werden vom Körper selbst durch zwei Enzyme gebildet. Vor allem die Leberzellen erzeugen Thiocyanat aus Cyaniden und anderen Produkten des Zellstoffwechsels. Der Rest muss von außen durch die Ernährung aufgenommen werden. Insbesondere Milchprodukte und vegetarische Ernährung führen dem Körper Thiocyanat zu. Moderne Hygiene kann bei Körper- und Haarwäsche jedoch Thiocyanat auswaschen. (Wikipedia sowie www.thiocyanat.de , Anja Fenske-Bengisch, Thiocyn GmbH, Stiftstr.30, 60313 Frankfurt)

Die Fähigkeit der Leber, gewisse Substanzen abzubauen, sowie ihre allgemeine Belastungsfähigkeit und damit auch ihre Reaktionsschnelligkeit nehmen mit dem Älterwerden ab. (Ali A. Siddiqui, MD, Thomas Jefferson University, msdmanuals, MSD SHARP & DOHME GmbH, Lindenplatz 1,85540 Haar) Die Fähigkeit und die Geschwindigkeit der körpereigenen Bereitstellung von Thiocyanat sinken dem entsprechend mit zunehmendem Lebensalter ab.

Es ist dar wesentliche Verdienst der erfinderischen Zusammensetzung von Thiocyanat und Arginin, dieses Defizit insbesondere bei älteren und hochbetagten Patienten in Extremsituationen wie der Infektion durch SARS-CoV-2 in kürzester Zeit und in der erforderlichen Menge auszugleichen.

## Patentansprüche

1. Antivirale thrombosehemmende Zusammensetzung zur Verwendung bei der Behandlung von Covid-19 mit Arginin als erstem Bestandteil
**dadurch gekennzeichnet, dass**
sie als einen zweiten Bestandteil Thiocyanate, auch Rhodanid genannt, enthält, und zwar als Kaliumthiocyanat oder als ein anderes Salz oder als ein Thiocyansäureester.

2. Antivirale thrombosehemmende Zusammensetzung zur Verwendung gemäß Anspruch 1,
**dadurch gekennzeichnet, dass**
als zweiter Bestandteil Natriumthiocyanat enthalten ist.

3. Antivirale thrombosehemmende Zusammensetzung zur Verwendung gemäß Anspruch 1 oder 2,
**dadurch gekennzeichnet, dass**
Arginin und Rhodanid jeweils den gleichen Anteil an der gesamten Masse der Zusammensetzung haben.

4. Antivirale thrombosehemmende Zusammensetzung zur Verwendung gemäß einem der vorhergehenden Ansprüche, **dadurch gekennzeichnet, dass** Arginin und Rhodanid jeweils einen Anteil von 0,3 % bis 3 %, bevorzugt 0,7 % bis 2%, besonders bevorzugt von 1,25 % an der gesamten Masse der Zusammensetzung haben.

5. Antivirale thrombosehemmende Zusammensetzung zur Verwendung gemäß einem der vorhergehenden Ansprüche, **dadurch gekennzeichnet, dass** sie mit 1,8-Cineol kombiniert wird.

6. Antivirale thrombosehemmende Zusammensetzung zur Verwendung gemäß Anspruch 5,
**dadurch gekennzeichnet, dass** das 1,8-Cineol in seinem natürlichen Vorkommen als Eukalyptusöl oder Pfefferminzöl oder in einem anderen Minzöl oder als Campher und/oder Weihrauch und/oder als ein anderes Terpen integriert wird.

7. Antivirale thrombosehemmende Zusammensetzung zur Verwendung gemäß einem der vorhergehenden Ansprüche, **dadurch gekennzeichnet, dass** sie Metallsalze wie CuSO₄ und/oder andere Kupfersalze und/oder Zink-Sulfat oder andere Zink-Salze und/oder Mangansulfat oder andere Mangan-Salze enthält.

8. Antivirale thrombosehemmende Zusammensetzung zur Verwendung gemäß einem der vorhergehenden Ansprüche, **dadurch gekennzeichnet, dass** Dimethylsulfoxid enthalten ist.

9. Antivirale thrombosehemmende Zusammensetzung zur Verwendung gemäß einem der vorhergehenden Ansprüche, **dadurch gekennzeichnet, dass** sie um Methylsulfonylmethan erweitert wird.

10. Antivirale thrombosehemmende Zusammensetzung zur Verwendung gemäß einem der vorhergehenden Ansprüche, **dadurch gekennzeichnet, dass** weitere Bestandteile NMDA-und ACE-Blocker oder ein anderes Amantadin sind.

## Claims

1. Antiviral thrombosis-inhibiting composition for use in the treatment of Covid-19 with arginine as a first component, **characterized in that** it contains as a second component thiocyanate, also called rhodanide, as potassium thiocyanate or as another salt or as a thiocyanic acid ester.

2. Antiviral thrombosis-inhibiting composition for use according to claim 1, **characterized in that** sodium thiocyanate is contained as a second component.

3. Antiviral thrombosis-inhibiting composition for use according to claim 1 or 2, **characterized in that** arginine and rhodanide each have the same proportion of the total mass of the composition.

4. Antiviral thrombosis-inhibiting composition for use according to any one of the preceding claims, **characterized in that** arginine and rhodanide each have a proportion of 0.3 % to 3 %, preferably 0.7 % to 2 %, particularly preferably 1.25 % of the total mass of the composition.

5. Antiviral thrombosis-inhibiting composition for use according to any one of the preceding claims, **characterized in that** it is combined with 1,8-cineole.

6. Antiviral thrombosis-inhibiting composition for use according to claim 5, **characterized in that** the 1,8-cineole is integrated in its naturally occurring state as eucalyptus oil or peppermint oil or in another oil of mint or as camphor and/or frankincense and/or as another terpene.

7. Antiviral thrombosis-inhibiting composition for use according to any one of the preceding claims, **characterized in that** it contains metal salts such as CuSQ₄ and/or other copper salts and/or zinc sulphate or other zinc salts and/or manganese sulphate or other manganese salts.

8. Antiviral thrombosis-inhibiting composition for use according to any one of the preceding claims, **characterized in that** dimethyl sulfoxide is contained.

9. Antiviral thrombosis-inhibiting composition for use according to any one of the preceding claims, **characterized in that** it is supplemented with methylsulfonylmethane.

10. Antiviral thrombosis-inhibiting composition for use according to any one of the preceding claims, **characterized in that** further components are NMDA and ACE blockers or another amantadine.

## Revendications

1. Composition antivirale inhibitrice de la thrombose à utiliser dans le traitement de la Covid-19 avec de l'arginine comme premier composant, **caractérisée par le fait qu'**elle contient comme second composant du thiocyanate, également appelé rhodanide, sous forme de thiocyanate de potassium ou d'un autre sel ou d'un ester d'acide thiocyanique.

2. Composition antivirale inhibitrice de la thrombose à utiliser selon la revendication 1, **caractérisée par le fait qu'**elle contient comme second composant du thiocyanate de sodium.

3. Composition antivirale inhibitrice de la thrombose à utiliser selon les revendications 1 ou 2, **caractérisée par le fait que** l'arginine et le rhodanide représentent chacun la même proportion de la masse totale de la composition.

4. Composition antivirale inhibitrice de la thrombose à utiliser selon l'une quelconque des revendications précédentes, **caractérisée en ce que** l'arginine et le rhodanide ont chacun une proportion de 0,3 % à 3 %, de préférence de 0,7 % à 2 %, de préférence encore de 1,25 % de la masse totale de la composition.

5. Composition antivirale inhibitrice de la thrombose à utiliser selon l'une quelconque des revendications précédentes, **caractérisée par le fait qu'**elle est associée au 1,8-cinéole.

6. Composition antivirale inhibitrice de la thrombose à utiliser selon la revendication 5, **caractérisée par le fait que** le 1,8-cinéole est intégré dans son état d'occurrence naturelle en tant qu'huile d'eucalyptus ou huile de menthe poivrée ou dans une autre huile de menthe ou en tant que camphre et/ou encens et/ou en tant qu'autre terpène.

7. Composition antivirale inhibitrice de la thrombose à utiliser selon l'une quelconque des revendications précédentes, **caractérisée par le fait qu'**elle contient des sels métalliques tels que CuSQ₄ et/ou d'autres sels de cuivre et/ou du sulfate de zinc ou d'autres sels de zinc et/ou du sulfate de manganèse ou d'autres sels de manganèse.

8. Composition antivirale inhibitrice de la thrombose à utiliser selon l'une quelconque des revendications précédentes, **caractérisée par le fait qu'**elle contient du sulfoxyde de diméthyle.

9. Composition antivirale inhibitrice de la thrombose à utiliser selon l'une quelconque des revendications précédentes, **caractérisée par le fait qu'**elle est complétée par du méthylsulfonylméthane.

10. Composition antivirale inhibitrice de la thrombose à utiliser selon l'une quelconque des revendications précédentes, **caractérisée par le fait que** les autres composants sont des bloqueurs NMDA et ACE ou une autre amantadine.
